Europäisches Patentamt

**European Patent Office**

**Office européen des brevets**

(19)

(11) Numéro de publication: **0 069 427**

**A2**

(12)

# DEMANDE DE BREVET EUROPEEN

(21) Numéro de dépôt: **82200823.1**

(22) Date de dépôt: **01.07.82**

(51) Int. Cl.³: **A 61 B 19/00**
**A 61 B 17/38**

(30) Priorité: **08.07.81 BE 2059251**

(43) Date de publication de la demande:
**12.01.83 Bulletin 83/2**

(84) Etats contractants désignés:
**AT CH DE FR GB IT LI LU NL SE**

(71) Demandeur: **FABRIQUE NATIONALE HERSTAL en abrégé FN Société Anonyme**

**B-4400 Herstal(BE)**

(72) Inventeur: **Waltregny, Alain J.**
**rue Louvrex 64**
**B-4000 Liege(BE)**

(72) Inventeur: **Kleinermann, Jacques G.**
**rue Graedt 34**
**B-4580 Aubel(BE)**

(74) Mandataire: **Bockstael, Daniel**
**M.F.J. Bockstael Arenbergstraat 13**
**B-2000 Anvers(BE)**

(54) **Dispositif stéréotaxique pour l'électro-coagulation des ganglions de Gasser.**

(57) Dispositif stéréotaxique pour l'électro-coagulation des ganglions de Gasser ou similaires, caractérisé par la combinaison d'un cadre fixe portant des moyens réglables d'immobilisation de la tête du patient dans une position telle que le plan contenant le point visé soit connu; d'un cadre mobile monté à rotation sur ledit cadre fixe et portant une glissière courbe; et d'un porte-électrode déplaçable le long de ladite glissière, l'agencement étant tel que tout déplacement du porte-électrode s'effectue à la surface d'une sphère centrée sur ledit point visé.

EP 0 069 427 A2

1

# Dispositif stéréotaxique pour l'électro-coagulation des ganglions de Gasser.

La présente invention concerne un dispositif stéréotaxique pour l'électro-coagulation des ganglions de Gasser.

Dans certains cas de névralgie du trijumeau, provoquant des douleurs répétées extrêmement brutales au niveau de la face, il est nécessaire de recourir à une intervention chirurgicale consistant à électrocoaguler le ganglion de Gasser en 4 choississant une cible en fonction de la topographie de la douleur périphérique.

Cette intervention nécessite l'introduction d'une aiguille-électrode au travers du trou ovale, ou foramen ovale. Elle est donc particulièrement délicate, de par la grande précision exigée. A la connaissance de la Demanderesse, il n'existe plus à ce jour d'appareil spécifiquement conçu pour la pratique de cette intervention.

Par analogie aux névralgies du trijumeau (nerf V), l'application du dispositif est étendue au traitement des névralgies d'un autre nerf crânien, le nerf glossopharyngien (nerf IX) par abord d'un autre orifice de la base du crâne à savoir le foramen jugulaire.

On connaît certes des appareils de stéréotaxie permettant l'immobilisation de la tête du patient tout en fournissant un système tri-axial orthogonal permettant de définir les coordonnées de n'importe quel point du cerveau (brevet belge n° 576.462). Leur emploi pour l'opération ici envisagée nécessite toutefois des adaptation particulières. Le but de l'invention est de fournir un dispositif facilitant considérablement l'opération.

A cet effet, un dispositif conforme à l'invention est constitué par la combinaison d'un cadre fixe portant des moyens réglables d'immobilisation de la tête du patient dans une position telle que le plan contenant le point visé soit connu; d'un cadre mobile monté à rotation sur ledit cadre fixe et portant une glissière courbe; et d'un porte-électrode déplaçable le long de ladite glissière, l'agencement étant tel que tout déplacement du porte-électrode s'effectue à la surface d'une sphère centrée sur ledit point visé.

Pour plus de clarté, un mode de réalisation de l'invention est décrit ci-après avec référence à la vue perspective annexée, donnée à titre illustratif et non restrictif.

Le dispositif illustré est donc constitué par la combinaison d'un cadre fixe généralement désigné par la référence 1 et d'un cadre mobile 2 monté à rotation sur une partie latérale 3, fixée de façon réglable par les dispositifs 4 sur ledit cadre 1.

Le cadre fixe 1 est constitué par une plaque de base 5 portant deux montants latéraux 6 réunis par une liaison supérieure 7. La plaque 5 porte deux colonnes 8 supportant chacune un tampon d'oreille 9 axialement réglable. Les centres de ces derniers sont substantiellement situés sur l'axe de rotation du cadre 2 sur le cadre 1, n'étant éloignés de cette droite que de quelques millimètres.

Chaque montant 6 porte un tampon de tête 10 axialement réglable, destinés à offrir appui aux zones mastoïdes de la tête du patient.

La liaison supérieure 7 porte un support nasal 11 axialement réglable.

Le cadre mobile 2 est constitué par deux flasques latérales
12 mutuellement reliées, en une extrémité, par une liaison
frontale 13. Cette dernière porte une glissière 14 courbée
en arc de cercle, sur laquelle peut être déplacé un porte-
électrode 15 dans lequel peut coulisser une aiguille-électrode 16 reliée à son appareil d'alimentation et de commande 17.

La partie latérale réglable 3 porte, en bout de ses deux
branches 18, une tige repère 19, des rainures 20 étant prévues par la fixation d'une plaque radiographique.

Le dispositif susdécrit est destiné à être utilisé conjointement avec un dispositif radiographique ou radioscopique de
type connu, de la manière suivante.

On immobilise la tête du patient dans le cadre fixe 1, au
moyen du support nasal 11 et des tampons 9 et 10.

Par rotation et élévation ou abaissement du cadre 2, on
amène l'axe du porte-électrode 15  à pointer dans le trou
ovale et on le centre ensuite dans ce dernier par un déplacement latéral adéquat le long de la glissière 14, ces opérations s'effectuant à l'aide de l'appareil de radiographie
susmentionné.

Ce dernier est également utilisé pour déterminer l'angle de
pénétration.  Le praticien enfonce ensuite l'électrode 16 en
travers du porte-électrode 15 jusqu'au point désiré.  Il vérifie la bonne pénétration par radiographie latérale.

On remarquera que le dispositif décrit est agencé de sorte
que tout se passe comme si le porte-électrode 15 se déplaçait sur une surface sphérique centrée sur le trou ovale du
patient, l'axe de l'électrode 16 passant par le centre dudit trou une fois les réglages préliminaires effectués.

Il est évident que différentes modifications peuvent être apportées au dispositif susdécrit sans pour autant sortir des limites de l'invention, plus particulièrement définies dans les revendications ci-après.

Revendications.

1.- Dispositif stéréotaxique pour l'électro-coagulation des ganglions de Gasser, ou similaires caractérisé par la combinaison d'un cadre fixe portant des moyens réglables d'immobilisation de la tête du patient dans une position telle que le plan contenant le point visé soit connu; d'un cadre mobile monté à rotation sur ledit cadre fixe et portant une glissière courbe; et d'un porte-électrode déplaçable le long de ladite glissière, l'agencement étant tel que tout déplacement du porte-électrode s'effectue à la surface d'une sphère centrée sur ledit point visé. Dans l'application au nerf glossopharyngien, la visée se fait sur le foramen jugulaire en lieu et place du trou ovale.

2.- Dispositif selon la revendication 1, caractérisé en ce que ledit cadre mobile est monté à rotation sur une partie latérale fixée de façon réglable en hauteur sur ledit cadre fixe.

3.- Dispositif selon la revendication 1, caractérisé en ce que lesdits moyens réglables d'immobilisation de la tête sont constitués d'une part par un support nasal destiné à engager l'arcade nasale du patient et, d'autre part, par deux tampons d'oreilles opposés.

4.- Dispositif selon la revendication 2, caractérisé en ce que les centres desdits tampons sont substantiellement situés sur l'axe de rotation du cadre mobile sur le cadre fixe.

5.- Dispositif selon la revendication 2, caractérisé en ce que lesdits moyens d'immobilisation comportent en outre deux tampons de tête réglables, destinés à être appliqués contre les zones mastoïdes de la tête du patient.

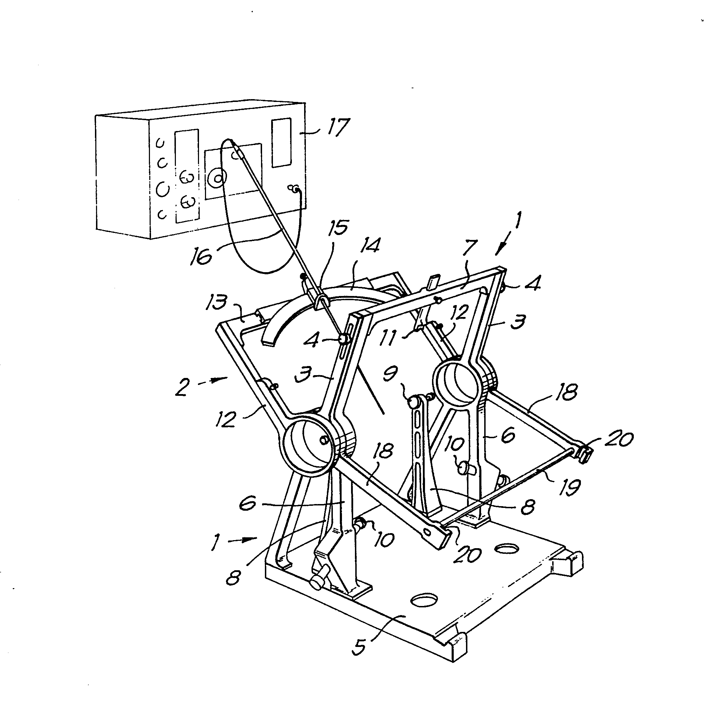

6.- Dispositif stéréotaxique pour l'électro-coagulation des ganglions de Gasser, substantiellement tel que décrit précédemment et illustré au dessin annexé.